# EUROPEAN PATENT APPLICATION

(11) **EP 4 385 466 A1**
(43) Date of publication of application: **19.06.2024**
(21) Application number: 22213574.1
(22) Date of filing: 14.12.2022
(51) Int. Cl.: A61F 2/958, A61F 2/91

(54) **MANUFACTURING METHOD FOR A SYSTEM COMPRISING A STENT AND A DELIVERY DEVICE HAVING A BALLOON**

(71) Applicant: BIOTRONIK AG, 8180 Bülach (CH)
(72) Inventor: Fargahi, Amir, 8180 Bülach (CH)
(74) Representative: Biotronik Corporate Services SE

(57) **Abstract**

The invention is directed to a manufacturing method for a system comprising a stent (30) and a delivery device (10, 110) having a balloon (19, 119) wherein the stent is crimped onto the balloon. The manufacturing method produces a system that exhibits higher initial displacement force and dislodgement force and comprises the following steps:
• Providing the stent (30) and the delivery device (10, 110) with a folded balloon (119),
• Crimping the stent (30) onto the material of said folded balloon (119) forming the outer shell surface, and
• Providing an ultrasonic treatment to the stent (30) after crimping and/or during crimping.

## Description

The invention relates to a manufacturing method for a system comprising a stent and a delivery device with a balloon and a system manufactured by such method.

Stents and similar implantable medical devices such as grafts, stent-grafts, vena cava filters, expandable frameworks, are radially expandable endoprostheses which are typically intravascular implants capable of being implanted transluminally and enlarged radially after being introduced percutaneously. In this document stent means any (conventional) stent or any radially expandable stent-like part or structure of above mentioned implantable medical devices.

Stents may be implanted in a variety of body lumens or vessels such as within the vascular system, urinary tracts, bile ducts, fallopian tubes, coronary vessels, secondary vessels, gastrointestinal tract, etc. They may be self-expanding, expanded by an internal radial force, such as when mounted on a balloon, or a combination of self-expanding and balloon expandable (hybrid expandable).

For expansion of a stent at a target location within a patient's body by the internal radial force, in many cases respective forces are generated by a balloon also referred to as balloon dilatation. Introduction into the patient's body and advancement of the stent towards the target location is provided by a system comprising a delivery device such as a balloon catheter with a folded balloon and a stent crimped onto the shell surface of the folded balloon. At the target location the stent is expanded by inflating the balloon through the delivery device and thereby secured at the target location. Then, the balloon is deflated and the delivery device is retracted from the patient's body.

Crimping is a procedural step during manufacturing of the system comprising the stent and the balloon catheter in which the stent is secured at the delivery device by radially compressing and plastically deforming the stent onto the balloon.

For reliable stenting procedure it is essential that the stent is securely fixed onto the surface of the balloon to avoid displacement or dislodgement of the stent from the balloon. ASTM-Standard F2394-07 defines an initial displacement force and a dislodgement force. The initial displacement force is the initial force that is required to displace the stent with respect to the balloon such that the displacement is a non-recoverable movement. The peak dislodgement force is a peak or maximum force required to completely dislodge the stent from the delivery device balloon during a stent securement test, wherein the dislodgement force will occur after or coincide with the initial displacement force.

Crimping procedures during manufacturing of the system comprising the delivery device and the stent often require several crimping and heating steps and usage of different crimping and heating machines which seems to be quite complicated and time consuming. Further, recoil of the stent may occur reducing the initial displacement force and the dislodgement force.

Accordingly, there is the need for a less complicated and time consuming procedure for manufacturing of the system comprising the delivery device and the stent leading to a secure fixation of the stent onto the balloon.

The above object is solved by a manufacturing method for a system comprising a stent and a delivery device as well as by such system.

In particular, the object is solved by a manufacturing method for a system comprising a stent and a delivery device having a balloon with the following steps:
- Providing the stent and the delivery device with a folded balloon,
- Crimping the stent onto the material of said folded balloon forming the outer shell surface, and
- Providing an ultrasonic treatment to the stent after crimping and/or during crimping.

The above mentioned stent comprises stents as defined above including radially expandable stent-like parts or structures of above described implantable medical devices, for example vascular stents (including stents for use in the heart and heart valve stents, e.g. mitral valve stent, pulmonary valve stent, aortic valve stent), bile duct stents, endoprostheses for the closure of persistent foramen ovale (PFO), stent grafts for the treatment of aneurysms, endoprostheses for the closure of an ASD (atrial septal defect) and similar prostheses in the area of hard and soft tissue. The stent has an openwork hollow cylindrical (tubular) and/or an openwork hollow cone-shaped structure having a longitudinal axis wherein the structure is open at both longitudinal ends. The structure may often be composed of a plurality of webs or struts.

The stent may be created by a method including cutting or etching a pre-defined design from a tubular stock, from a flat sheet which is cut or etched and which is subsequently rolled or from one or more interwoven wires or braids. The stent may usually adopt an expanded state and a compressed (non-expanded) state in which the stent has a smaller outer diameter than in the expanded state. The stent is provided for manufacturing of the system in an expanded state.

Further, the delivery device (e.g. a catheter) is provided having a balloon fixed at the distal end of the delivery device. The delivery device is configured such that it introduces the stent into the patient's body and advances it to the target location for treatment. The balloon is configured such that it can be used to dilate the stent accommodated at its outer surface at the target location. For that, the balloon is inflated causing an expansion and/or unfolding of the balloon so that it provides a radial force to the stent for dilation. The delivery device may comprise a tube-like form at its distal end. The delivery device may be a balloon catheter. Catheters are tubes or tubular members of various diameters that can be inserted into the patient's body cavity to be treated. Prior the catheter, a guide wire and, if necessary, a guide catheter may be used and may first be inserted into the organ or vessel to be treated prior to the catheter. The catheter may then be inserted and advanced along the guide wire to the site of the organ or vessel to be treated, so that the stent arranged on the catheter is placed at the target location of the organ or vessel. The stent is then dilated using the radial force provided by the balloon. In particular, the radial force is provided by balloon inflation using a fluid (e.g. saline, contrast medium). The catheter is then removed. At the same time or subsequently, the guidewire is also withdrawn from the organ or vessel, if applicable. A balloon catheter may comprise an inner tubular member (inner shaft) and an outer tubular member (outer shaft) and the balloon provided at its distal end. The balloon may be fixed to the distal end of the inner tubular member at its distal end and to the distal end of the outer tubular member at its proximal end.

The balloon is folded at the distal end of the delivery device such that its outer diameter is reduced. In the unfolded and inflated state the balloon material has a greater diameter than the distal end of the delivery device. For example, the balloon may be folded in one flap or more than one flap and the flap(s) may be tightly laid around the distal end of the delivery device, for example spirally or helically. Thereby, the balloon adopts a form comparable to a hollow cylinder wherein the inner diameter of this hollow cylinder corresponds to the outer diameter of the distal end of the delivery device at the position of the folded balloon. The inner form of the folded balloon adapts to the outer form of the delivery device at the position of the folded balloon, e.g. to the form of the inner tubular member.

The crimping step comprises the reduction of the outer diameter of the stent and fixing it onto the outer shell surface of the folded balloon. Accordingly, by crimping, the inner diameter and form of the stent adapts to the outer diameter and form of the folded balloon. Prior to crimping, the stent is placed around the folded balloon. In particular, the stent is slided from the distal end of the catheter over the folded balloon. For crimping the delivery device and the stent is placed into a crimping apparatus having, for example, an iris type mechanism as a crimping tool. The iris type mechanism may apply a radial force to the stent (e.g., it may close crimping jaws or blades onto the stent) so that it reduces its diameter until it reaches a pre-defined dimension and/or a pre-defined maximum crimping force. Afterwards, the system comprising the delivery device having the balloon and the stent crimped onto the outer shell surface of the folded balloon may be removed from the crimping apparatus.

After a crimping step and/or during crimping the ultrasonic treatment may be provided to the system (of said folded balloon with said crimped stent), in particular to the stent. Another crimping step may follow. The ultrasonic treatment applies vibrations, for example radial vibrations to the stent. The ultrasonic treatment may be provided for 10 seconds to 5 minutes, for example, for 30 seconds to 2 minutes.

The inventive manufacturing method, in particular its ultrasonic treatment provides a deeper pressing of the stent struts into the balloon material forming the outer shell surface due to the frictional heat generated by the vibrations of the stent struts along the surface of the balloon material. Therein, the material forming the shell surface is the folded material located externally. Accordingly, an optimal mechanical form locking is provided for the stent struts by the respective abutting balloon material thereby increasing the initial displacement force and the dislodgement force. Additionally, the manufacturing time is decreased by the above method. In particular, the drive unit for ultrasonic treatment may be connected to different vibrators (such as the tube) that transmit the ultrasonic vibration directly to the stent.

Alternatively, the step of providing the stent and the delivery device comprises providing the stent and the delivery device with a (unfolded) balloon, and the manufacturing method further comprises the step of folding the balloon such that the balloon adopts a form comparable to a hollow cylinder having a longitudinal axis.

In one embodiment, the ultrasonic vibrations may be provided to the stent by a tube of an ultrasonic device that can be vibrated. In this case, prior the ultrasonic treatment, the system is introduced into the tube such that the crimped stent is fully surrounded by the tube. The inner diameter of the tube corresponds to the outer diameter of the crimped stent so that the tube abuts the inner surface of the tube. For the ultrasonic treatment, the tube is driven such that it provides ultrasonic vibrations, for example radial vibrations, to the abutting stent surface. Alternatively, during crimping, the crimping apparatus may provide ultrasonic vibration to the crimping tool, e.g., to crimping jaws or blades. Such tube realizes the ultrasonic treatment in an easy and cost effective way. The tube or the crimping tool is driven by a respective drive unit that generates the respective vibrations and transmits it to the tube or the crimping tool. Further, the ultrasonic device or crimping apparatus may comprise one tube or crimping tool, respectively, or more than one tube or crimping tool, respectively. In one embodiment, the two or more than two tubes comprise a single base station with a drive unit, wherein the driving unit drives simultaneously the two or more than two tubes.

In one embodiment of the method, an optimal range of ultrasonic treatment is provided with a vibration frequency from 10 kHz to 50 kHz, for example, from 20 kHz to 35 kHz.

In one embodiment of the method, a heat treatment is simultaneously provided with the ultrasonic treatment and/or prior the ultrasonic treatment. By the heat treatment, the balloon material is, for example, heated above the glass temperature of the respective balloon material, for example at a temperature between 30 °C and 150 °C, e.g. between 50 °C and 120 °C. The heat treatment may be provided during a time period from 5 seconds to 5 minutes. The used temperature depends on the material of the balloon and the material of the stent since the stent material at the surface of the balloon material is heated, too. For a drug eluting stent comprising a drug at the surface of the stent that is released in a pre-defined way over a pre-defined time period.

In one embodiment of the method, an overpressure is provided to the inner lumen of the folded balloon during ultrasonic treatment, for example by inflating the balloon with air. The overpressure (e.g. of 10 bar to 25 bar) ensures that the balloon material provides a counter force so that the stent structure (e.g. the stent struts) has good contact to the balloon material. Accordingly, the development of frictional heat is improved.

In one embodiment of the method, the stent is pre-crimped prior the crimping onto the shell surface of the folded balloon. The pre-crimping is advantageous because it yields to a stable and precise positioning of the stent on the folded balloon.

The above object is further solved by a system comprising a stent and a delivery system having a balloon manufactured by the above described method. The system has the advantages discussed with regard to the above method. The system is characterized by a balloon having recesses in which the stent struts are located after the final crimping step and ultrasonic treatment. The recesses provide the form locking fixation of the stent onto the surface of the balloon.

The stent comprises or consists of, for example, at least one material of the group comprising stainless steel, titanium alloy, cobalt chromium alloy, magnesium alloy or plastics, e.g. PLA, PLLA etc.

The balloon comprises or consists of, for example, at least one material of the group comprising thermoplastic materials such as Polyether block amide (PEBA), Polyamid (PA), Polyethylene Terephthalate (PET), Polyetheretherketon (PEEK) etc.

The stent material and/or the balloon material may comprise a coating material, for example a medicinal product.

The present invention will now be described in further detail with reference to the accompanying schematic drawings, wherein
- Fig. 1: shows an embodiment of a balloon catheter in a side view and partly cut-out representation,
- Fig. 2: depicts a stent in an expanded state and in a perspective side view,
- Fig. 3: shows a distal end of a system comprising a balloon catheter and the crimped stent of Fig. 2 in a side view,
- Fig. 4: shows an ultrasonic treatment device in a front view,
- Fig. 5: depicts the device of Fig. 4 and the system of Fig. 3 in a front view, and

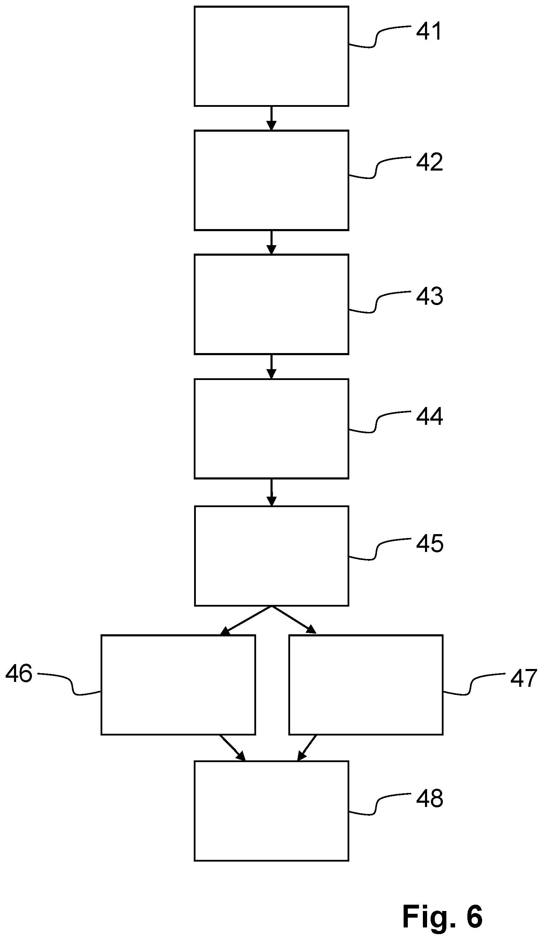

- Fig. 6: shows a flowchart of the manufacturing method of the system of Fig. 3.

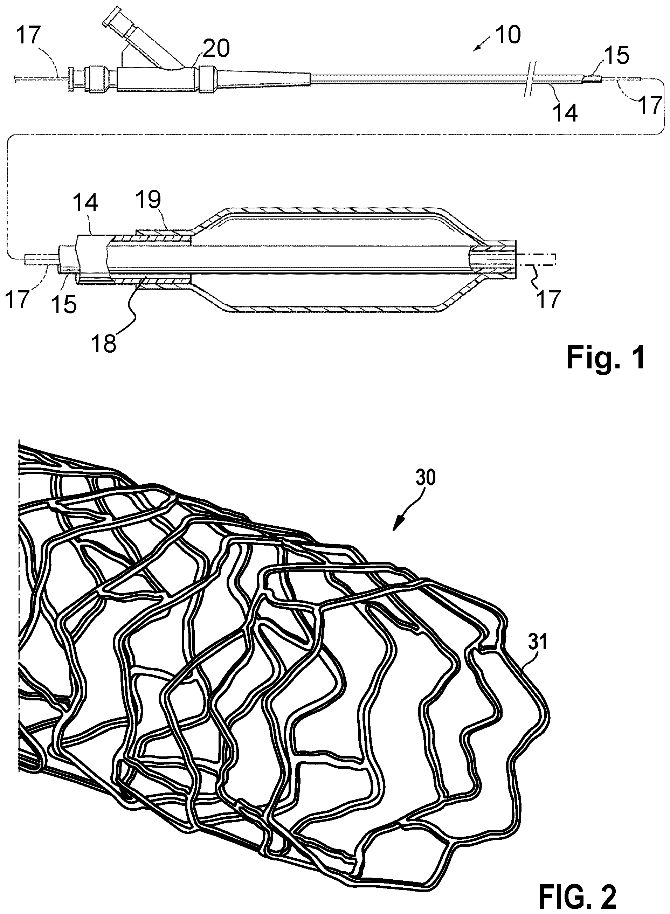

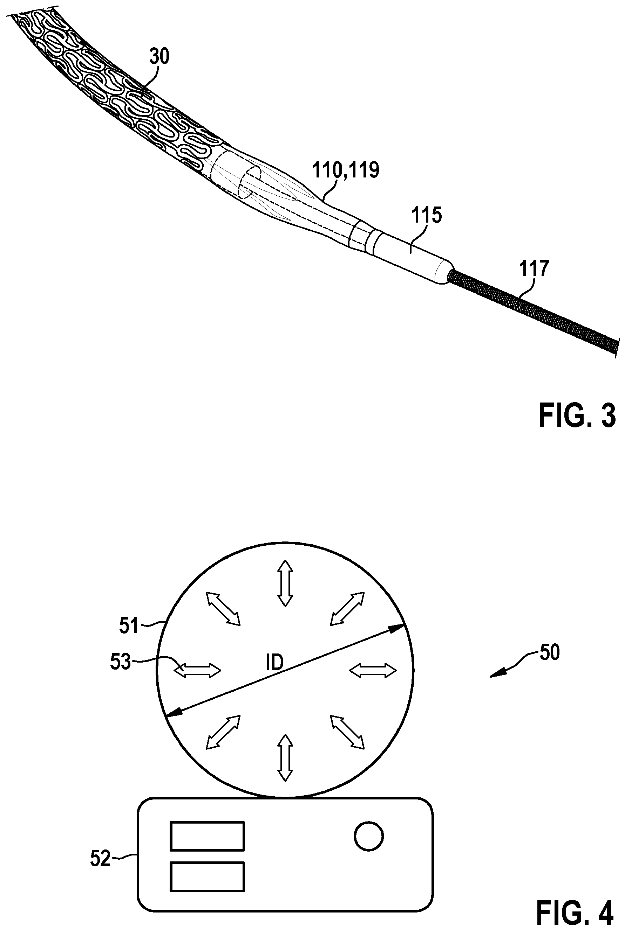

Fig. 1 illustrates a delivery device in the form of a catheter 10 with an inflated balloon 19. The catheter 10 comprises an elongated catheter shaft having a proximal end and a distal end, an outer tubular member 14 and an inner tubular member 15. The inner tubular member defines a guidewire lumen adapted to slidingly receive a guidewire 17. The coaxial relationship between the outer tubular member 14 and the inner tubular member defines an inflation lumen 18. The inflatable balloon 19 is disposed on the distal shaft section, wherein the proximal end of the balloon 19 is sealingly secured to the distal end of the outer tubular member 14 and the distal end of the balloon 19 is sealingly secured to the distal end of the inner tubular member 15. The balloon 19 may be consist of Grilamid Polyamid-12 L25 or Pebax. An adapter 20 at the proximal end of the catheter shaft is configured to provide access to the guidewire lumen and to direct inflation fluid into inflation lumen 18. The distal end of the catheter 10 may be advanced to a target location within the patient's body for release and dilation of a stent 30 as exemplarily shown in Fig. 2 in an expanded state. The stent has a plurality of struts 31 and may consist, for example, of a cobalt chromium alloy. Fig. 3 depicts a system comprising the stent of Fig. 2 and a balloon catheter 110 that has basically the same construction as the one shown in Fig. 1. The balloon catheter 110 has a longer inner tubular member 115 at its distal end compared with the stent shown in Fig. 1. It extends from the distal end of the balloon 119. Additionally, the guidewire 117 extends from the distal end of the inner tubular member 115. Prior introduction and advancement, the balloon 119 is folded and the stent 30 crimped onto the shell surface of the balloon 119 to reduce the outer diameter of the system as shown in Fig. 3. At the target location, the balloon is configured to be inflated through the inflation lumen thereby generating a radial force to dilate the stent 30. After this procedure, the balloon is configured to be deflated to release the stent 30 from the balloon and to be withdrawn from the patient's body.

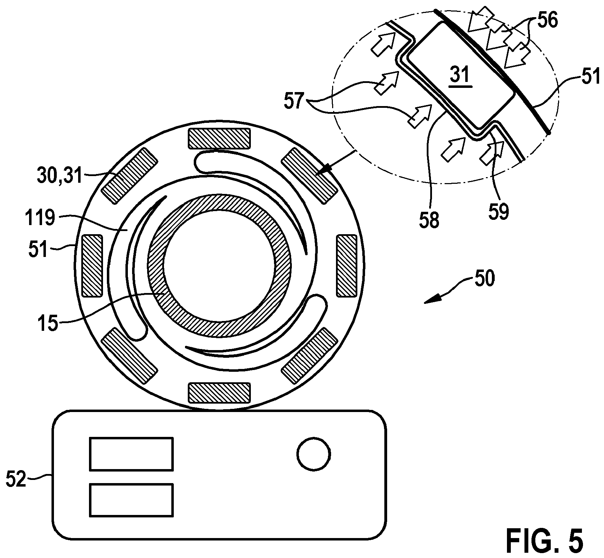

With regard to the flow chart, the manufacturing method for the system shown in Fig. 3 is explained in the following. In the first step 41 the stent 30 and the balloon catheter 10, 110 is provided as shown in Fig. 1 and 2. Then, in step 42 the balloon 19, 119 is deflated and folded such that the balloon 119 adopts a form comparable to a hollow cylinder having a longitudinal axis which is the longitudinal axis of the inner tubular member 15. In one embodiment, the material of the balloon 119 may be folded in flaps and the flaps may be laid one above the other and at the same time wound around the inner tubular member 15 as one may derive from the front view of Fig. 5. After that, the stent 30 is reduced in diameter by crimping and is crimped onto the outer shell surface of the folded balloon 119. Crimping may be provided in two steps, for example, a pre-crimping step to a first (greater) diameter and a crimping step to a second (smaller) diameter. Between the pre-crimping step and the crimping step the pre-crimped stent 30 may be moved in longitudinal direction along the shell surface of the folded balloon to correctly position the stent onto the shell surface of the folded balloon 119.

In step 44 the system comprising the balloon catheter 10, 110 with the folded balloon 119 and the crimped stent 30 is introduced into a tube 51 of an ultrasonic device 50 which is shown in Fig. 4 without the system and in Fig. 5 with the system. The ultrasonic device 50 comprises a base station 52 with a control unit and a drive unit that drives the tube 51 such that it vibrates in radial direction (represented by arrows 53 of Fig. 4). The inner diameter ID (see Fig. 4) of the tube 51 corresponds to the outer diameter of the crimped stent 30 so that the stent struts 31 abut the inner surface of the tube 51. The longitudinal axis of the tube 51 is parallel or identical to the longitudinal axis of the inner tubular member 15 of the balloon catheter 110 when correctly positioned within the tube 51.

Afterwards, in step 45, the system, in particular the balloon 119 is heated, for example at a temperature between 50 °C and 110 °C, for 10 seconds. For that the ultrasonic device 50 may be equipped with an electric resistance heating element embedded within the material of the tube 51. Due to this heating the balloon material becomes plastically deformable.

Further, in the next step 46, the balloon is slightly inflated so that the internal pressure within the balloon 119 is, for example, 15 bar. At the same time, in step 47, the tube 51 provides vibrations at a frequency between 20 kHz and 35 kHz to the stent - the ultrasonic treatment for about 60 seconds. During the ultrasonic treatment, there are radial forces provided by the vibrating tube 51 (see arrows 56 in Fig. 5) and radial forces (represented by arrows 57) to the balloon material into the opposite direction provided by its internal pressure. Thereby, at the contact area 58 of the stent strut 31 of the stent 30 and the balloon 119 frictional heat is generated and the stent strut form is more deeply pressed into the balloon material (see recess 59). Accordingly, an optimal mechanical form locking is provided between the stent struts 31 and the balloon material thereby increasing the initial displacement force and the dislodgement force.

After the ultrasonic treatment, in step 48, the balloon is deflated and removed from the tube 51.

It shall be emphasized that the above method not only assures a more reliable fixation of the stent onto the balloon of the catheter but is also a very fast and cost-effective procedure.

## Claims

1. A manufacturing method for a system comprising a stent (30) and a delivery device (10, 110) having a balloon (19, 119) with the following steps:
• Providing the stent (30) and the delivery device (10, 110) with a folded balloon (119),
• Crimping the stent (30) onto the material of said folded balloon (119) forming the outer shell surface, and
• Providing an ultrasonic treatment to the stent (30) after crimping and/or during crimping.

2. The manufacturing method according to claim 1, wherein the ultrasonic treatment comprises radial vibrations transmitted to said stent (30).

3. The manufacturing method according to any one of the previous claims, wherein the ultrasonic vibrations are transmitted to said stent by a tube (51) surrounding the stent (30).

4. The manufacturing method according to any one of the previous claims, wherein the ultrasonic treatment is provided with a vibration frequency from 10 kHz to 50 kHz.

5. The manufacturing method according to any one of the previous claims, wherein a heat treatment is simultaneously provided with the ultrasonic treatment and/or prior the ultrasonic treatment.

6. The manufacturing method according to claim 5, wherein the heat treatment is provided at a temperature of 50 °C to 150 °C.

7. The manufacturing method according to any one of the previous claims, wherein an overpressure is provided to the inner lumen of the folded balloon (119) during ultrasonic treatment.

8. The manufacturing method according to any one of the previous claims, wherein the stent (30) is pre-crimped prior the crimping onto the shell surface of the folded balloon (119).

9. System comprising a stent (30) and a delivery system (10, 110) having a balloon (19, 119) manufactured by the method according to any one of the previous claims.
